# EUROPEAN PATENT SPECIFICATION

(11) **EP 1 631 593 B1**
(45) Date of publication and mention of the grant of the patent: **17.08.2011**
(21) Application number: 04733834.8
(22) Date of filing: 19.05.2004
(51) Int. Cl.: C08B 31/04, C08B 37/14, D21H 17/29

(54) **A PROCESS FOR THE PREPARATION OF CARNITINE ESTERS AND THEIR USE**
VERFAHREN ZUR HERSTELLUNG VON ESTERN DES CARNITINS UND IHRE VERWENDUNG.
PROCEDE DE PREPARATION D'ESTERS DE CARNITINE ET LEUR UTILISATION

(30) Priority: 21.05.2003 FI 20030769; 15.08.2003 FI 20031157
(43) Date of publication of application: 08.03.2006
(73) Proprietor: BASF SE, 67056 Ludwigshafen (DE)
(72) Inventor: LAINE, Aki, FI-21200 Raisio (FI); KOSKIMIES, Salme, FI-00850 Helsinki (FI); KÄKI, Jouko, FI-20300 Turku (FI); LUTTIKHEDDE, Hendrik, Jan, Gerrit, FI-21260 Raisio (FI); NURMI, Kari, FI-21200 Raisio (FI); YLI-KAUHALUOMA, Jari, FI-00710 Helsinki (FI)
(74) Representative: Hollatz, Christian
(86) International application number: PCT/FI2004/000302
(87) International publication number: WO 2004/104049

(56) References cited:
- WO-A-00/15669
- DE-A- 10 027 393
- DE-A- 10 133 200
- US-A- 4 439 438

## Description

### Summary of the invention

This invention covers a method for the preparation of carnitine esters of starch and other hydroxy polymers, and their use in several applications of industry, for example as an additive in the manufacture of paper. The esterification of a hydroxy polymer, preferably starch, with β-lactone of carnitine is most feasibly carried out in an aqueous slurry. The carnitine esters of starch are considered as more physiologically acceptable and biodegradable than traditional cationic starch ethers.

### Background of the invention

Starch is a renewable and economical raw material and the third most used component by weight in paper industry. The main role of starch is to improve the strength of paper. Starch is also used as an adhesive in surface sizing and as a binder in coating formulations. The bonding of starch to cellulosic fiber is generally improved by addition of cationic substituents to the starch backbone. The positively charged cationic starch, containing amino or ammonium groups, has a strong affinity for negatively charged surfaces and particles i.e. cellulosic fibers and mineral pigments.

Cationic starches are also used in textile industry to improve the textile feel of the fabric. In waste water treatment, the use of cationic starches improves the retention of anionic impurities in the flocculation processes.

The use of low molecular weight cationic starches in cosmetics and the treatment of a keratin-containing substrate is disclosed in US patent 6365140. Another cosmetic treatment composition comprising cationic starch betainate has been described in patent publication WO 02/07684, which also covers a cosmetic treatment method for keratinous matter and use for washing skin.

Several methods have been developed for the cationization of starch. The cationization is generally carried out by etherification of starch with 2,3-epoxypropyl trimethyl ammonium chloride or 3-chloro-2-hydroxypropyl trimethyl ammonium chloride in an alkaline aqueous slurry or in a dry process. The common cationization reagent can give undesirable reaction by-products.

As the common cationic starches are etherified with cationic reagents, more biodegradable and physiologically acceptable cationic starches should have functional groups esterified on the starch backbone. However, the most methods for preparation of starch esters, especially cationic starch esters, are unfeasible in large-scale production.

The generally known methods of preparing carboxylic acid esters of starch involve the use of acid chlorides or anhydrides in organic solvents such as pyridine or 1,4-dioxane. Patent publication WO 00/15669 illustrates the esterification of starch using acid chloride of betaine in 1,4-dioxane and pyridine. Patent FR 2805270 concerns novel types of cationic polymers and polymeric matrices, degradable in the organism, and with controlled rate of degradation, useful as such or as vehicles for different compounds, in particular molecules with biological activity. FR 2805270 also describes a method for producing said polymers and matrices from maltodextrins and acid chlorides of betaines in pyridine and dimethyl formamide (DMF).

The use of undesired and relatively expensive solvents and reagents generate both environmental load and high price for starch esters and may leave traces of harmful substances in final products. Therefore, the general esterification methods do not fulfill the requirements for the high-volume and low-cost starch esters, especially when the application of the starch ester may be involved in food products, cosmetics or pharmaceuticals.

The production of anthranilic acid ester of starch and its use as a paper retention aid has been described in the patents NL 6717509, US 3499886, US 3511830, US 3513156 and US 3620913. The esterification of starch is performed using isatoic anhydride in an organic solvent or an aqueous slurry. Isatoic anhydride (i.e. N-carboxy anhydride of anthranilic acid) is generally prepared from anthranilic acid and phosgene. The hydrolysis product shows biological activity.

A retention aid for chemical pulp prepared by derivatization of dialdehyde starch with betaine hydrazide has been described in Tappi 44, 1962, 750. However, the thus formed hydrazones of starch are harmful and their preparation is complex and unfeasible.

Starch esters have been derived from lactones, but neither synthesis of cationic nor nitrogen-containing starch esters from β-lactones have been reported. Esterification and etherification of starch with aliphatic β-lactones is described in patent GB 675793. The patent covers the use of β-propiolactone, β-butyrolactone, β-isobutyrolactone, βvalerolactone and β-isovalerolactone in preparation of starch solutions of improved stability. Alkaline reaction conditions are claimed to yield starch esters, whereas neutral or acidic conditions are claimed to produce starch ethers of the aliphatic β-hydroxy acids. A similar method is described in patent US 3580906, where starch levulinates are prepared using α-angelica lactone (i.e. 4-hydroxy-3-pentenoic acid γ-lactone) in alkaline starch solutions or in absence of a liquid solvent.

In patent publication WO 95/25750, starch is graft co-polymerized with ε-caprolactone without any solvent at high temperatures of 100-200 °C. Thus grafted hydrophobic starch esters can be used as hot melt adhesives and impermeable coatings for paper.

A process for preparation of cationic poly- and oligoesters of lactic acid and carnitine from di-lactide and carnitine β-lactone is described in patent publication DE 10027393. The use of thus prepared carnitine polyesters as cosmetic and hair treatment agents is disclosed.

### Detailed description of the invention

This invention covers a novel method for the preparation of hydroxy polymer esters of carnitine. Preferred hydroxy polymers for use in the method according to the invention are selected from the group consisting of unmodified or modified starch, cellulose, chitosan, guar gum, xanthan, polyvinyl alcohol and mixtures thereof. An especially preferred hydroxy polymer is starch. The cationic starch esters prepared according to the invention can replace conventional cationic starches in several applications. The invented process does not include undesired substances, and the starch esters prepared according to the invention are more biodegradable than the traditional cationic starch ethers.

L-carnitine (i.e. (R)-3-hydroxy-4-(trimethyl ammonium)butanoate) is a natural amino acid, which has an important role in energy production of cells. A part of biologically essential L-carnitine is gained in the nutrition, especially from meats and animal foods, and L-carnitine is also synthesized in the body from amino acids lysine and methionine. In pharmacotherapy, L-carnitine is well known to help in the treatment of angina pectoris and heart pain induced by physical stress.

In the invented process, carnitine ester of a hydroxy polymer, preferably starch, is prepared by reacting β-lactone of carnitine with said hydroxy polymer, preferably starch, in an aqueous alkaline medium. In the process, carnitine is covalently bound via an ester bond to at least part of the hydroxyl groups of the hydroxy polymer. The β-lactone of carnitine is preferably a racemic or enantiomerically pure β-lactone of carnitine chloride, bromide, iodide, mesylate, tartrate, fumarate, formiate, acetate or propionate. Both D and L forms, as well as DL form, can be used. The pure L-carnitine esters are preferred in the applications, where a complete biocompatibility is required, whereas the racemic products are more suitable for low cost applications such as paper production.

The reaction can be performed in an organic solvent, such as 1,4-dioxane or dimethyl sulfoxide (DMSO), or in the absence of solvents, but the most preferable reaction medium is an aqueous hydroxy polymer slurry, such as aqueous starch slurry.

In the aqueous esterification, the hydrolysis of formed hydroxy polymer ester by alkali catalyst competes with the esterification reaction. However, excellent reaction efficiencies (RE), even 90% or more, can be achieved by carefully selecting the reaction conditions. The aqueous esterification is preferably carried out at a pH of 7-10 by using reaction times of for example 0.5-10 h and temperatures of 10-50 °C, even 80 °C. After the esterification, starch carnitates are neutralized or acidified to stabilize esters against hydrolysis. The reaction time may vary depending on the type of the reactor, the reaction temperature and the choice of reagents. At higher temperatures, shorter reaction times are needed but higher temperatures also accelerate the hydrolysis of the desired carnitine ester. The progress of the esterification reaction should be carefully monitored, e.g. by NMR analysis or by studying the viscosity or solubility of the reaction mixture.

For improved yields in an aqueous esterification, high hydroxy polymer concentrations and the use of low viscosity hydroxy polymers as raw material are recommended. In the preparation process of starch esters of carnitine, starch concentrations may vary for example from 1 to 40%, preferably between 20 and 30%. When the degree of substitution (DS) raises above 0.1, dilution of the starch slurry is usually required to maintain the miscibility and the pH control of the reaction. For other hydroxy polymers, hydroxy polymer concentrations should be as high as practicable, depending on the reactor design and the processability of the hydroxy polymer used. A person skilled in the art having knowledge of the reagents, equipment and analysis methods to be used can easily select and optimize the reaction conditions.

The invented process comprises solely of natural, physiologically acceptable and economical raw materials, and the products are fully biodegradable. The esterification process can be feasibly performed using readily available apparatuses used for example in the preparation of traditional cationic starch ethers.

The hydroxy polymer esters prepared according to the invention are suitable for paper and paperboard manufacture, e.g. as wet end additives and in sizing applications. When neither undesired raw materials nor solvents are used, the biodegradable and physiologically acceptable hydroxy polymer carnititates, prepared according to the invention, are applicable especially as additives of food, paper or paperboad or cardboard, and in cosmetic and pharmaceutical compositions. The invention is also directed to the use of hydroxy polymer esters of carnitine in waste water treatment and as a textile sizing agent.

Consequently, cationic starch carnitates can replace traditional cationic starches in applications such as paper manufacture, waste water treatment and textile sizing. In addition, starch carnitates have special potential in pharmaceutical applications, cosmetics and food products. Cationic starch carnitate has a strong affinity on ceratinous material such as skin and hair and can therefore be used to improve various cosmetic compositions. Carnitine esters of guar gum or starch are evident substitutes for conventional guar hydroxypropyltrimonium chloride, e.g. in hair and skin care applications. Hair conditioning compositions containing monomeric carnitine derivates have been recently published (WO 02/074265 and WO 03/005980). In addition, hydroxy polymer carnitates can be used as polyelectrolyte surfactants in applications such as moisturizing creams and antiperspirants. As cationic polymers posses anti-microbial properties, hydroxy polymer carnitates could be used as preservatives of low toxicity or as antibacterial agents of deodorants. In gene therapy, hydroxy polymer carnitates could be used as non-toxic cationic polymer vectors, which carry therapeutic DNA sequences into target cells. Hydroxy polymer carnitates can also enhance the composition of tablets and matrices of slow release drugs. Natural guar gum is generally consumed to treat high cholesterol levels, because natural guar gum functions as a weak anion exchange resin and thereby binds some of the cholic acid in the bowels. However, the anion exchanger character of guar gum can be improved by esterification with biocompatible carnitine, which may upgrade the effect of guar gum or other digestible hydroxy polymers in the medication of high cholesterol levels.

It will be appreciated that the essence of the present invention can be incorporated in the form of variety of embodiments, only a few of which are disclosed herein. It will be apparent for the skilled person that other embodiments exist and do not depart from the spirit of the invention. Thus the described embodiments should not be construed as restrictive. For example although starch is preferred material for the process, also some other hydroxy polymer, such as cellulose, chitosan, guar gum, xanthan or polyvinyl alcohol, could be used, and starch or other hydroxy polymer might also be modified, e.g. thinned (i.e. acid hydrolyzed or oxidized).

### EXAMPLES

### Example 1. Esterification of starch with DL-carnitine bromide β-lactone

Oxidized potato starch (70.0 g; 1.0 eq.) was slurried in 230 ml of water. DL-carnitine bromide β-lactone (26.1 g; 0.27 eq.) was added. The slurry was stirred at 20 °C and 10% K₂CO₃ solution was added to maintain pH at 8.5 (±0.2). After the reaction time of 30 minutes the mixture thickened and 240 ml of water was added to sustain the miscibility of the solution. After total reaction time of 2 h the pH was adjusted to 4 with 1 M HCl. Cationized starch was precipitated with 2200 ml of ethanol and the solution was decanted. Starch was dissolved in 900 ml of water, precipitated with 3500 ml of ethanol and the solution was decanted. Starch was suspended in 500 ml of ethanol, collected by filtration and dried in vacuum. The esterification yielded 65.8 g of pale yellowish carnitine ester of starch (DS 0.17; RE 64%).

### Example 2. Esterification of starch with DL-carnitine bromide β-lactone

Native potato starch (35.0 g; 1.0 eq.) was slurried in 75 ml of water. DL-carnitine bromide β-lactone (2.42 g; 0.05 eq.) was added. The slurry was stirred at 20 °C and 10% K₂CO₃ solution was added to maintain pH at 9.0 (±0.2). After total reaction time of 1.5 h the pH was adjusted to 5 with 1 M HCl. 200 ml of ethanol was added and starch was collected by filtration. The product was purified twice by slurrying in 100 ml of water and precipitating with 200 ml of ethanol. Starch was collected by filtration and dried in vacuum. The esterification yielded 36.1 g of pale carnitine ester of starch (DS 0.04; RE 80%).

### Example 3. Esterification of guar gum with DL-carnitine bromide β-lactone

Guar gum (2.00 g; 1.0 eq.) was dissolved in 130 ml of water. DL-carnitine bromide βlactone (0.83 g; 0.30 eq.) was added. The slurry was stirred at 20 °C and 10% K₂CO₃ solution was added to maintain pH at 8.0 (±0.2). After total reaction time of 5 h the pH was adjusted to 5 with 1 M HCl. Guar gum was precipitated with 300 ml of ethanol and collected by filtration. Guar gum was dissolved in 150 ml of water, precipitated with 400 ml of ethanol and collected by filtration. The esterification yielded 1.28 g of pale DL-carnitine ester of guar gum (DS 0.07; RE 23%).

### Example 4. Esterification of hydroxypropylcellulose with L-carnitine chloride β-lactone

Hydroxypropylcellulose (4.00 g; 1.0 eq.; MW 100 000; molecular substitution 4.9) was dissolved in 200 ml of water. L-carnitine chloride β-lactone (0.16 g; 0.10 eq.) was added. The slurry was stirred at 20 °C and 10% NaOH solution was added to maintain the pH at 8.0 (±0.2). After total reaction time of 4 h the pH was adjusted to 5 with 1 M HCl. Water was removed by evaporation. The esterification yielded L-carnitine ester of hydroxypropylcellulose (DS 0.06; RE 60%).

### Example 5. Esterification of starch with L-carnitine chloride β-lactone in DMSO

Oxidized potato starch (5.0 g; 1.0 eq.) was dissolved in 50 ml of dry DMSO by heating at 120 °C. The solution was cooled to 20 °C and L-carnitine chloride β-lactone (4.44 g; 0.80 eq.) was added. The slurry was stirred at 40 °C and few drops of pyridine were added to adjust the alkalinity of the solution. After total reaction time of 3 h the solution was acidified with HCl. The cationized starch was precipitated with diethyl ether and collected by filtration. The esterification yielded 6.64 g of carnitine ester of starch (DS 0.61; RE 76%).

### Example 6. Retention of carnitine ester of starch on cellulose fibers

The adsorption tendency of carnitine ester of starch on cellulose was examined by DDJ (Dynamic Drainage Jar). DDJ test was done according to Tappi-standard T261 cm-90.

Starches in the test:
1. Carnitine ester of starch. (Starch prepared according to example 2). DS: 0.04 Viscosity at 60 °C (conc. 5%, jet-cooked at 130 °C): 195 mPas
2. Cationic wet-end starch (Raisamyl 135). DS: 0.035. Viscosity at 60 °C (conc. 5%, jet-cooked at 130 °C): 172 mPas
3. Cationic wet-end starch (Raisamyl 145). DS: 0.045. Viscosity at 60 °C (conc. 5%, jet-cooked at 130 °C): 215 mPas

Furnish in the test:
Birch cell: 50%
Pine cell: 50%
Consistency: 2.0%
pH: 5.9
Schopper & Riegler value: 20

### Procedure:

Starches were slurried in water. Concentration of the slurries was 6%. Sample of 400 ml of each slurry was taken and jet-cooked (cooking with steam) with pilot jet-cooker. Starches were diluted into concentration of 5% for viscosity measurement and then finally into concentration of 1%. Starches were dosed into the furnish, agitated for 2 min and diluted with tap water into consistency of 0.6%. Each test sample was tested with DDJ-apparatus (100 rpm) and the filtrates were collected and analysed. Starch concentration and cationic demand was determined from the filtrates.

| Starch | Dosage | CD | Conc. of starch in water | Starch retention |
|---|---|---|---|---|
| | (kg/tn) | µmol/l | (mg/l) | (%) |
| Reference | 0 | -30 | 0.0 | - |
| 1. Carnitine ester of starch | 5 | -27 | 3.1 | 90 |
| | 10 | -20 | 6.5 | 89 |
| 2. Raisamyl 135 | 5 | -25 | 3.6 | 88 |
| | 10 | -21 | 7.0 | 88 |
| 3. Raisamyl 145 | 5 | -27 | 3.9 | 87 |
| | 10 | -14 | 4.5 | 93 |

The results of the test show clearly that the starch concentrations in the filtrates are equal and thus the retention level of starch carnitine ester is the same as for conventional wet end starches. The impact of starch carnitine ester on cationic demand is equal compared to reference starches.

## Claims

1. A process for the preparation of a hydroxy polymer ester of carnitine, **characterized in that** a hydroxy polymer is reacted with a β-lactone of carnitine.

2. The process according to claim 1, **characterized in that** the β-lactone of carnitine is βlactone of carnitine chloride, bromide, iodide, mesylate, tartrate, fumarate, formiate, acetate or propionate.

3. The process according to claim 1 or 2, **characterized in that** the hydroxy polymer is selected from the group consisting of unmodified or modified starch, cellulose, chitosan, guar gum, xanthan, polyvinyl alcohol and mixtures thereof, preferably starch.

4. The process according to any one of claims 1 to 3, **characterized in that** the reaction is carried out in an aqueous alkaline medium.

5. The process according to any one of claims 1 to 4, **characterized in that** the reaction is carried out at a temperature of 10-80°C.

6. The process according to any one of claims 1 to 5, **characterized in that** the hydroxy polymer is starch and its concentration is from 1 to 40%, preferably between 20 and 30%.

7. The use of the hydroxy polymer ester of carnitine, preferably starch ester, produced according to any one of claims 1 to 6, in the manufacture of paper, paperboard or cardboard, in waste water treatment, as an additive of food, as a textile sizing agent or in cosmetic or pharmaceutical compositions.

8. The use of a hydroxy polymer ester, preferably starch ester, of carnitine in the manufacture of paper, paperboard or cardboard, in waste water treatment, as a textile sizing agent, as an additive of food, or in cosmetic or pharmaceutical treatment compositions.

## Patentansprüche

1. Verfahren zur Herstellung eines Hydroxypolymeresters von Carnitin, **dadurch gekennzeichnet, dass** ein Hxdroxypolymer mit einem β-Lacton von Carnitin umgesetzt wird.

2. Verfahren gemäß Anspruch 1, **dadurch gekennzeichnet, dass** das β-Lacton von Carnitin β-Lacton von Carnitinchlorid, -bromid, -iodid, -mesylat, -tatrat, -fumarat, -formiat, -acetat oder propionat ist.

3. Verfahren gemäß Anspruch 1 oder 2, **dadurch gekennzeichnet, dass** das Hydroxypolymer ausgewählt ist aus der Gruppe, bestehend aus nicht modifizierter oder modifizierter Stärke, Cellulose, Chitosan, Guargummi, Xanthan, Polyvinylalkohol und Mischungen davon, bevorzugt Stärke.

4. Verfahren gemäß einem der Ansprüche 1 bis 3, **dadurch gekennzeichnet, dass** die Reaktion in einem wässrigen alkalischen Medium durchgeführt wird.

5. Verfahren gemäß einem der Ansprüche 1 bis 4, **dadurch gekennzeichnet, dass** die Reaktion bei einer Temperatur von 10-80 °C durchgeführt wird.

6. Verfahren gemäß einem der Ansprüche 1 bis 5, **dadurch gekennzeichnet, dass** das Hydroxypolymer Stärke ist und seine Konzentration von 1-40 %, bevorzugt zwischen 20 und 30 % beträgt.

7. Verwendung des Hydroxypolymeresters von Carnitin, bevorzugt Stärkeester, hergestellt gemäß einem der Ansprüche 1 bis 6, bei der Herstellung von Papier, Pappe oder Karton, in der Abwasserbehandlung, als Zusatzstoff bei Lebensmitteln, als ein textiles Schlichtemittel oder in kosmetischen oder pharmazeutischen Zusammensetzungen.

8. Verwendung eines Hydroxypolymeresters, bevorzugt Stärkeesters, von Carnitin zur Herstellung von Papier, Pappe oder Karton, in der Abwasserbehandlung, als ein textiles Schlichtemittel, als Zusatzstoff bei Lebensmitteln oder in kosmetischen oder pharmazeutischen Zusammensetzungen.

## Revendications

1. Procédé de préparation d'un ester d'hydroxypolymère de carnitine, **caractérisé en ce qu'**un hydroxypolymère est mis à réagir avec une β-lactone de carnitine.

2. Procédé selon la revendication 1, **caractérisé en ce que** la β-lactone de carnitine est le chlorure, le bromure, l'iodure, le mésylate, le tartrate, le fumarate, le formiate, l'acétate ou le propionate de β-lactone de carnitine.

3. Procédé selon la revendication 1 ou 2, **caractérisé en ce que** l'hydroxypolymère est choisi dans le groupe constitué par un amidon modifié ou non modifié, une cellulose, un chitosane, une gomme de guar, un xanthane, un alcool polyvinylique et des mélanges de ceux-ci, de préférence un amidon.

4. Procédé selon l'une quelconque des revendications 1 à 3, **caractérisé en ce que** la réaction est réalisée dans un milieu alcalin aqueux.

5. Procédé selon l'une quelconque des revendications 1 à 4, **caractérisé en ce que** la réaction est réalisée à une température de 10 à 80 °C.

6. Procédé selon l'une quelconque des revendications 1 à 5, **caractérisé en ce que** l'hydroxypolymère est un amidon et sa concentration est comprise dans la plage allant de 1 à 40 %, de préférence de 20 à 30 %.

7. Utilisation de l'ester d'hydroxypolymère de carnitine, de préférence de l'ester d'amidon, produit selon l'une quelconque des revendications 1 à 6, dans la fabrication de papier, de carton ou de carton blanchi, dans le traitement des eaux usées, en tant qu'additif alimentaire, en tant qu'agent d'ensimage textile ou dans des compositions cosmétiques ou pharmaceutiques.

8. Utilisation d'un ester d'hydroxypolymère, de préférence un ester d'amidon, de carnitine dans la fabrication de papier, de carton ou de carton blanchi, dans le traitement des eaux usées, en tant qu'additif alimentaire, en tant qu'agent d'ensimage textile ou dans des compositions cosmétiques ou pharmaceutiques.
